# EUROPEAN PATENT SPECIFICATION

(11) **EP 1 299 135 B1**
(45) Date of publication and mention of the grant of the patent: **20.09.2006**
(21) Application number: 00943991.0
(22) Date of filing: 04.07.2000
(51) Int. Cl.: A61L 15/38, A61K 38/48

(54) **WOUND DRESSING COMPRISING TERRILYTIN AS THERAPEUTICALLY ACTIVE AGENT**
WUNDVERBAND MIT TERRILYTIN ALS AKTIVEM THERAPEUTISCHEM AGENS
PANSEMENT COMPRENANT LA TERRILYTIN COMME AGENT THERAPEUTIQUE ACTIF

(43) Date of publication of application: 09.04.2003
(73) Proprietor: Frohwitter, Bernhard, 81679 München (DE)
(72) Inventor: FILATOV, Vladimir Nikolaevich, Moscow 125284 (RU); RYLTSEV, Vladimir Valentiovich, Moscow 109029 (RU)
(74) Representative: Savic, Bojan
(86) International application number: PCT/EP2000/006278
(87) International publication number: WO 2002/002155

(56) References cited:
- GB-A- 2 240 040
- DATABASE CHEMABS [Online] CHEMICAL ABSTRACTS SERVICE, COLUMBUS, OHIO, US; VIRNIK, A.D. ET AL: "Immobilization of enzymes on grafted cellulose copolymers containing ionizable groups" retrieved from STN Database accession no. 121:173907 XP002161987 & CELLUL. CHEM. TECHNOL. (1993), 27(5), 477-88 ,
- DATABASE CHEMABS [Online] CHEMICAL ABSTRACTS SERVICE, COLUMBUS, OHIO, US; VIRNIK, A. D. ET AL: "Preparation and characterization of films with combined biological effects" retrieved from STN Database accession no. 128:58953 XP002161988 & PRIKL. BIOKHIM. MIKROBIOL. (1997), 33(4), 428-432 ,
- DATABASE CHEMABS [Online] CHEMICAL ABSTRACTS SERVICE, COLUMBUS, OHIO, US; VIRNIK, A. D. ET AL: "Obtaining of fibrous materials containing both immobilized proteolytic enzyme and anti-microbial substance, and investigation of their properties" retrieved from STN Database accession no. 126:154423 XP002161989 & PRIKL. BIOKHIM. MIKROBIOL. (1996), 32(6), 615-619 ,
- DATABASE CHEMABS [Online] CHEMICAL ABSTRACTS SERVICE, COLUMBUS, OHIO, US; YUDANOVA, T. N. ET AL: "Properties of enzyme-containing fibrous biomedical materials" retrieved from STN Database accession no. 118:132079 XP002161990 & IZV. VYSSH. UCHEBN. ZAVED., KHIM. KHIM. TEKHNOL. (1992), 35(9), 95-9 ,
- DATABASE CHEMABS [Online] CHEMICAL ABSTRACTS SERVICE, COLUMBUS, OHIO, US; YUDANOVA, T. N. ET AL: "Stability of enzyme-containing fibrous materials in storage after.gamma.-sterilization" retrieved from STN Database accession no. 115:239429 XP002161991 & IZV. VYSSH. UCHEBN. ZAVED., KHIM. KHIM. TEKHNOL. (1991), 34(7), 100-3 ,
- DATABASE CHEMABS [Online] CHEMICAL ABSTRACTS SERVICE, COLUMBUS, OHIO, US; GOSTISHCHEV, V. K. ET AL: "Experimental and clinical use of various forms of immobilized proteinases and their inhibitors" retrieved from STN Database accession no. 103:172040 XP002161992 & VOPR. MED. KHIM. (1985), 31(4), 21-4 ,
- DATABASE CHEMABS [Online] CHEMICAL ABSTRACTS SERVICE, COLUMBUS, OHIO, US; YUDANOVA, T. I. ET AL: "Continuous procedures for immobilization of terrilytin on cellulosic fibrous materials" retrieved from STN Database accession no. 104:182435 XP002161993 & BIOTEKHNOLOGIYA (1985), (6), 98-102 ,
- DATABASE CHEMABS [Online] CHEMICAL ABSTRACTS SERVICE, COLUMBUS, OHIO, US; YUDANOVA, T. N. ET AL: "Manufacture of a modified cellulose fiober containing the immobilized proteolytic enzyme terrilytin" retrieved from STN Database accession no. 105:139568 XP002161994 & IZV. VYSSH. UCHEBN. ZAVED., TEKHNOL. TEKST. PROM-STI. (1986), (3), 75-7 ,
- DATABASE CHEMABS [Online] CHEMICAL ABSTRACTS SERVICE, COLUMBUS, OHIO, US; GOSTISHCHEV, V. K. ET AL: "A comparison of the efficacies of native and immobilized proteinases in treatment of infected wounds" retrieved from STN Database accession no. 107:51979 XP002161995 & VOEN.-MED. ZH. (1987), (3), 32-4 ,

## Description

### Field of the invention

This invention pertains to the field of medicine and, in particular, to wound dressings with terrilytin as therapeutically active agent attached thereto. More particularly, in one aspect this invention relates to wound dressings having terrilytin as therapeutically active agent attached thereto in order to improve the healing process of pyo-necrotic wounds. In a further aspect, the invention relates to a method for manufacturing said wound dressings is disclosed.

### Background of the invention

The attachment of biologically active material to a carrier is well known in the art not only from the literature (including patent literature) but also from the use of commercially available products. U.S. Pat. No. 3,705,084 describes a flow-through enzyme reactor comprising an enzyme adsorbed on a polymeric surface and cross-linked thereon by a bifunctional cross-linking agent. U.S. Pat. No. 4,237,229 teaches a method according to which a protein, coenzyme or antibiotic is attached to polyurethane polymers. U.S. Pat. No. 3,791,927 discloses a process for preparing a water-insoluble bound protein entrapped within cells of a self-supporting reticulated cellular material, e.g. polyurethane foam.

Proteins attached to cellulose-based carriers, e.g. carriers made of cellulosic fibers, are also known. U.S. Pat. No. 4,013,514 discloses a flow-through reactor using derivatives of active enzymes covalently attached to a fibrous dialdehyde cellulose such as cotton. By passing a suitable substrate solution through such a reactor containing active enzyme, large quantities of the substrate will be converted into desired products. The reactor can be used for proteolysis, conversion of starch to glucose, hydrolysis of pectins, and the like.

Of particular interest is the use of therapeutically active agent for the preparation of a medicament for topical application to wounds. Grzybowski et al. reported the use of an antibacterial dressing for infected wounds (*J. Biomed. Mater. Res.* 36: 163-166, 1997). This dressing is composed of a collagen membrane and a collagen sponge with an antibiotic containing layer in between. U.S. Pat. No. 5,836,970 teaches the use of a wound dressing comprising a combination of chitosan and alginate in order to achieve a bacteriostatic and hemostatic effect. The wound dressing is provided in the form of a powder, film, gel, foam or mixtures thereof. According to the teaching of U.S. Pat. No. 5,098,417 a wound dressing is disclosed for systematic administration of physiologically or biologically active agent adsorbed thereon by ionic interaction.

RU 2,131,268 discloses a dressing material with curative properties based on medicinal cellulose-derived gauze. Trypsin alone or in combination with insulin is chemically attached to dialdehyde groups which have been introduced by oxidation into cellulose used as carrier material. It was shown that, in principle, dressings with trypsin attached thereon could be effective in the treatment of pyo-necrotic wounds of most etiologies. However, under these conditions trypsin has only a reduced therapeutic effect. First, the quantity of attached trypsin is very low (0.25 mg per gram of the carrier) and secondly, the degree of oxidation of cellulosic moities is very high (2 %). Most of the enzyme turns out to be covalently bound to the carrier in a way which leads to reduced therapeutic efficacy. The 3-D structure of the enzyme is distorted, the conformational flexibility restrained and the relative activity of the chemically bound enzyme diminished when compared to native enzyme. Frequent changing of the dressing is required in order to achieve the therapeutic effect.

RU 2,142,818 describes a so-called Filatov-Ryltsev dressing, a partly oxidized medicinal gauze treated with trypsin alone, or trypsin in combination with lysozyme or insulin. Nevertheless, the therapeutic effect, is reduced due to the low degree of oxidation of cellulosic moities (0.32-0.48%). As a result most of the enzyme attached to the carrier gets rapidly washed into the wound discharge.

GB 2 240 040 discloses a wound-dressing comprising enzymes, like proteolytic enzymes, which are covalently linked to the carrier.

Moreover trypsin, a serine protease which is widely used in medicinal treatment, is relatively expensive due to the labor- and time-consuming steps required for its isolation. Since it is usually isolated from the bovine pancreas, the application of trypsin in the field of wound dressings is also hampered with respect to consistent quality of the protease and by the possibility of transmitting diseases.

The expense to trypsin utilization has made it necessary to compromise between the costs of trypsin and the therapeutic efficiency, i.e. in view of its physiological and/or biological activity. Thus, a need continues to exist for a wound dressing which is more effective than those disclosed in the prior art and yet allows a reduction of the amount of a therapeutically active agent attached to the carrier in order to minimize production costs of the wound dressing.

### Objects of the invention

Accordingly, it is an object of the present invention to provide a wound dressing with a therapeutically active agent attached thereto which exhibits an improved healing process of pyo-necrotic wounds.

Another object of the present invention is to provide a wound dressing which exerts a prolonged therapeutic effect on pyo-necrotic wounds thus reducing the necessity to exchange the wound dressing as frequently as in the case of prior art wound dressings.

A further object of the present invention is to provide a wound dressing with the properties of improved and longer lasting wound healing, which may be produced inexpensively, without contamination from animal sources and with assured control of quality.

These objects are achieved by a wound dressing as defined in claim 1. Upon further study of the specification and claims of the invention, further objects and advantages of this invention will become apparent to those skilled in the art.

### Summary of the invention

The present invention provides a wound dressing according to claim 1 and a method of manufacturing a wound dressing according to claim 7.

The present invention relates to wound dressings as well as methods which utilize such wound dressings for the treatment of pyo-necrotic wounds. Wound dressings are disclosed comprising a cellulose-based carrier having a therapeutically active agent attached thereto in order to improve the healing process of pyo-necrotic wounds.

The invention teaches the attachment of the therapeutically active agent or agents to the carrier in effective quantities, with an attachment mechanism and/or orientation which maintains therapeutic activity. Preferred therapeutic agents are proteinaceous, proteolytic enzymes being particularly preferred agents.

The proteolytic enzyme terrilytin is used as therapeutic agent. Terrilytin not only surpasses the properties of trypsin when used for the treatment of pyo-necrotic wounds but is cost-effective as well. Terrilytin is used in combination with at least one additional protein which has been selected in such a way that synergy in their therapeutic effect occurs. A combination of terrilytin, lysozyme and another protein, especially insulin, is preferred.

The invention also provides a method for the preparation of said wound dressings using a carrier in the form of a fabric or cloth. Preferred methods are those wherein the carrier is activated prior to attachment of the therapeutic agent, and those characterized in that the agent attached to said carrier is present in at least three therapeutically active fractions (termed herein the therapeutically active "triple system").

Accordingly, a wound dressing is claimed comprising terrilytin as therapeutically active agent and a cellulose-based carrier, wherein the therapeutically active agent comprises the enzyme terrilytin and at least one additional protein is attached to said carrier. Moreover, a wound dressing is claimed wherein the wound dressing comprises at least a therapeutically active system comprising three fractions of therapeutically active agent, namely an adsorbed fraction, a "stabilized fraction" and an "immobilized fraction". The wound dressings of this invention and the therapeutically active triple system comprising the therapeutically active agent are claimed for use for the treatment of pyo-necrotic wounds.

Furthermore, a method for manufacturing wound dressings according to the invention is claimed which comprises the steps of activating said carrier by oxidizing an initial medicinal gauze with sodium periodate in an aqueous solution, preparing separate protein solutions in buffered solutions and combining the prepared protein solutions to a combined protein solution before adding the combined protein solution to said oxidized medicinal gauze attachment or alternatively treating said oxidized medicinal gauze sequentially with separate protein solutions, allowing the protein solution to react for a period of time sufficient to reach a predetermined level of attachment, removing the final product from the protein solution, washing and drying it, characterized in that the cellulose-based carrier has an oxidation degree of approximately 0.5-1.5 %.

Moreover, the use of the enzyme terrilytin for the manufacture of a medicament for the treatment of pyo-necrotic wounds is claimed wherein the medicament are wound dressings as defined in the claims.

### Detailed description of the invention

Within the context of the invention, the term "cellulose―based carrier" is understood to be a water―insoluble substrate in form of a fabric or cloth, at least a portion of which is cellulosic. The terms "cellulose" and "cellulosic" both include cellulose itself and water-insoluble derivatives thereof. The "carrier" to be treated by the method for the preparation of the wound dressing according to the present invention may be any suitable non-woven, woven, knitted or knotted fabric, at least a portion of which is cellulosic. Any suitable derivative of cellulose, e.g. organic esters of cellulose, such as cellulose formate or cellulose acetate, and cellulose ethers, such as methyl cellulose or ethyl cellulose, may be employed.

The term "therapeutically active" should be understood as having a physiological and/or biological activity. Within the scope of the present invention, "therapeutically active agent" should be understood as any-protein or-protein derivative such as lysozyme or insulin in combination with the enzyme terrilytin. The "therapeutically active agent" could comprise one or more proteins or protein derivatives which has been isolated from natural sources (i.e. plant, animal or microbial in origin) and/or one more proteins or protein derivatives isolated from hosts genetically engineered according to recombinant DNA methods known in the art.

A method is disclosed for the preparation of said wound dressing comprising an activation step of the cellulose-based carrier followed by the attachment of the therapeutically active agent thereto. Within the context of the invention, "activation" is understood as the chemical or physical modification of the cellulosic portion of the carrier in order to improve the attachment of the therapeutically active agent of the present invention. A representative example for the activation is the oxidation of at least some glucoside units to dialdehyde groups (i.e. dialdehyde cellulose, DAC). Within the scope of the present invention, the term "attached" is understood as the permanent or temporary loading of the therapeutically active agent of the present invention onto the cellulose-based carrier, based on any kind of interaction such as, but is not limited to, ionic interaction, van der Waals-interaction, dipole-dipole interaction, hydrogen bonds and covalent bonds.

The enzyme terrilytin (CAS RN 37338-91-3) has, like trypsin, proteolytic activity but offers several additional advantages. Terrilytin is produced by the fungus *Aspergillus terricola* and was shown to be a complex comprising three proteolytic enzymes. Two of the proteases (proteases I and II) are of the group of serine proteases as judged by their physico-chemical and enzymatic characteristics and might be considered as two sub-types of the same enzyme. Protease III shows characteristics specific for metalloproteases of microbial origin (Selezneva and Bol'shakova, *Prikl. Biokhim. Mikrobiol.* 22: 3-11, 1986). The medicinal use of terrilytin, e.g. its fibrinolytic and anti-inflammatory properties, has been shown (M. D. Mashkovsky, *Therapeutic Remedies,* Vol. 2, pp. 60-61, Meditsina Publishing House, Moscow, 1993). The proteolytic activity of terrilytin is the sum of the activities of all subunits. The term "proteolytic unit" describes quantitatively the ability of proteolytic cleavage of peptide bonds in proteins. For more details see Byelov et al., *Khimikofarmatsevtichesky Zhurnal* 12: 101-103, 1992.

Terrilytin offers several advantages over trypsin with respect to manufacturing wound dressings for the treatment of pyo-necrotic wounds. Firstly, terrilytin can be obtained on a commercial scale with consistent quality using mircobiological techniques such as industrial fermentation. Secondly, enzymes of microbial origin, in contrast to those from animal sources like trypsin, generally tend to have a higher specific activity due to an increased degree of purity of the enzyme in the preparation. Thirdly, in contrast to trypsin, the possibility of transmitting disease is substantially reduced due to the microbiological origin of terrilytin: there is no associated hazardous contamination of the product by animal viruses. Fourthly, as terrilytin also comprises a metalloprotease, the range of possible substrates is broader in comparison to that of trypsin. Fifthly, the protease subunits constituting terrilytin appear to show synergistic effects and mutually to enhance the proteolytic activities of individual subunits. Sixthly, the autolytic properties of trypsin are well known in the art: the activity of trypsin is rapidly reduced in aqueous media, a property which is not known for terrilytin. This is highly advantageous with respect to the preparation and manipulation of terrilytin on an industrial scale during the manufacturing of the wound dressings of the invention.

A further embodiment of the present invention provides a cost-effective dressing with excellent therapeutic activities on the basis of partly oxidized cellulose to which two or more proteins are attached. The invention discloses the co-attachment of the enzyme terrilytin or a derivative thereof in combination with at least one additional protein. In particular, insulin or hen egg lysozyme are preferred, especially recombinantly produced versions of these proteins. The components of the therapeutically active agent of the present invention are preferably attached in equimolar proportions.

The additional protein(s) used in combination with terrilytin or a derivative thereof may be obtained from any suitable source, e.g. plants, animal, or microbial sources. Usually, these proteins can conveniently be obtained from microorganisms including bacteria, yeast, fungi and the like by using well-known fermentation methods. Many of the proteins are commercially available. A typical protein mixture used in the present invention consists of terrilytin in combination with lysozyme and insulin providing the attachment of three therapeutically active proteins to the cellulose-based carrier as the result of simultaneous and/or sequential immobilization thereon.

The additional protein(s) co-attached with terrilytin, and their respective concentrations have been selected in the present invention in such a way that an unanticipated synergy occurs in their therapeutic effect. For example, the time to reduce or even completely remove debris naturally present in the wound (required for wound healing) decreases from 5 days for lysozyme treatment to 3 days for trypsin treatment, 2 ½ days for combined trypsin and lysozyme treatment to - as in the case of the present invention - only 2 days for combined terrilytin and lysozyme treatment. This results in a further advantage of the present invention: the reduced necessity to exchange the applied wound dressing during a single course of treatment, namely from 3 napkins in the case of wound dresssings comprising the cellulose-based carrier with trpysin alone or trypsin in combination with other proteins, down to only 1 napkin when using the carrier terrilytin alone or terrilytin in combination with other proteins.

It is not obvious for people skilled in the field of wound treatment to combine a proteolytic enzyme like terrilytin with a protein like the hormone insulin. Experimentation, however, shows a surprising synergistic effect of this combination by accelerating the cleaning process of a wound as judged from the degradation of debris and healing of trophic ulcers. Wound dressings manufactured according to the invention which carry both terrilytin and insulin attached thereon exert curative effects not only during the first stage of wound healing, but also during the regeneration of lost soft tissue. The terrilytin-insulin wound dressing strongly improves cellular proliferation, granulation growth and epithelisation. A complete cleaning of the wound from debris is observed after two days, versus three days using techniques disclosed in the prior art.

This surprising synergism is helpful for the treatment of pyo-necrotic wounds of various etiologies, such as but not limited to proctitis, paraproctitis, otitis, decubitus sores, trophic ulcers, burns, frost bite, gunshot injuries, incised wounds, gangrene and keloid scars. These types of wounds may frequently lead to the amputation of an extremity. The present invention as disclosed herein, however, offers a new approach to their treatment.

For the production of the dressing according to the present invention, a cellulose-based carrier is activated by introducing reactive groups, in particular dialdehyde groups, into the cellulosic portion of the carrier to which the therapeutically active agent is subsequently attached. For example, dialdehyde cellulose (DAC) is produced by exposing medicinal gauze to periodic acid. The oxidative conversion to of polysaccharides to dialdehyde cellulose using periodic acid as an oxidizing agent is well known in the art, e.g. see U.S. Pat. No. 4,082,743. In a second step, attachment of therapeutically active agent is accomplished by passing the activated carrier through an aqueous solution containing the desired proteins.

The oxidation step is carried out until the degree of maximal oxidation the cellulose―based carrier has reached the range of approximately 0.5-1.5 %. A preferred degree of oxidation lies in the range of approximately 0.9-1.1 %. The degree of oxidation of the cellulose-based carrier is assessed by a procedure well-known in the field (e.g. Byelov et al., *Khimikofarmatsevtichesky Zhurnal* 12, 101-103, 1992).

The periodate oxidation product is treated with terrilytin in combination with insulin and/or lysozyme, or another protein, preferably in a phosphate buffer at a pH of 3.0 to 6.0, preferably approximately 4.4; and at a protein concentration of in the range from 0.001 to approximately 5 wt.-%, preferably approximately 0.01 wt.-% and a bath ratio of 2.5 to 5.5, preferably 4. The bath ratio or liquid factor is equal to the weight of cellulose-based carrier treated, divided by the weight of the solution. Thus one weight unit of said carrier is immersed in preferably 4 weight units of the appropriate protein solution. In practice, 1 kg of activated cellulose-based carrier is immersed in approximately 4 L of the aqueous solution.

The amount of protein present in the three therapeutically active fractions being attached to the carrier can be determined as the difference between the amount of protein in the aqueous solution before and after the procedure. Both concentrations are measured spectrophotometrically using a calibration curve (Ryltsev and Virnik, *Antibiot. Khimioter* 3: 202-205, 1989). Following this protocol, usually 0.4 mg of protein is attached per 1 g of activated carrier. This corresponds to a proteolytic activity of approximately 0.1 PU/g or more in the case of terrilytin, a bacteriolytic activity of approximately 10 U or more in the case of lysozyme and an activity of approximately 27 IU/g or more in the case of insulin.

The precise activity of the protein employed as starting material depends on the exact method of preparation and is not critical to the present invention provided that the attached protein has the desired therapeutic activity. Various analytical methods are known in the art to determine the activity of proteins. Protease activity of proteolytic proteins can be determined, for example, by well known casein digestion methods.

Attachment of terrilytin in combination with other proteins according to the invention can be carried out in an aqueous medium at a temperature and pH conditions which do not cause inactivation of the protein. The activated cellulose-based carrier may be treated sequentially or simultaneously with different protein solutions (in the latter case protein solutions are mixed immediately prior to the treatment). Temperatures above approximately 65° C should generally be avoided. The present process is readily carried out at ambient temperature using a buffered aqueous solution of the proteins. The temperature of choice depends, however, on the particular protein or mixture of proteins used. Usually the temperature ranges from approximately -5° C to approximately 30° C. A temperature in the range from approximately 10° C to approximately 25° C is preferred. Each of the protein concentrations can vary in the range from 0.01 mg/ml to approximately 50 mg/ml, preferably in the range from 0.1 mg/ml to approximately 2 mg/ml each.

The present invention provides a method which allows an optimal correlation between the degree of oxidation of a cellulose-based carrier and the amount of the therapeutically active agent attached thereto. According to the invention the chosen oxidation degree of the activated carrier and the amount of the chosen proteins lead to the presence of at least three fractions ("triple system") of the therapeutically active agent attached to the carrier: an adsorbed fraction, a "stabilized fraction" and an "immobilized fraction".

The three fractions can be characterized as follows: The first fraction comprises a therapeutically effective agent attached to the activated cellulose-based carrier by adsorption only. The second fraction, so-called "stabilized fraction", is formed by the activated cellulose-based carrier being degraded by a hydrolytic process during exposure to the microenvironment of a wound. In this context it should be noted that the properties of the cellulose-based carrier are very different to those of the activated cellulose-based carrier (DAC). While the former is a chemically inert and water-insoluble substance, the later is chemically active and can, at least in part, dissolve in water due to hydrolysis. During the exposure of medicinal gauze to periodic acid, dialdehyde moieties are unevenly introduced. Areas with a higher concentration of dialdehyde groups seem to be more prone to hydrolysis. The immobilization of a protein following the method disclosed in this invention does not alter the patterns of hydrolytic degradation. As a result cellulose oligomers with proteins attached thereto are produced. Areas with a lower concentration of dialdehyde groups are more resistant to hydrolysis so that it takes longer until these areas are degraded to cellulose oligomers. This latter fraction is called "immobilized fraction". The kinetics of the hydrolytic process are described in Ryltsev and Virnik, *Antibiot. Khimioter* 3: 202-205, 1989.

Taking all this together, the activity of the therapeutically active agent attached to the wound dressing is superior to the corresponding native protein in solutions as shown by clinical experiments. The "triple system" provides a shock dose based on an adsorbed therapeutically active agent, a minimum therapeutic dose based on a therapeutically active agent present in the "immobilized fraction" and a dose based on a therapeutically active agent present in the "stabilized fraction" which further contributes to the healing effect of the wound dressing disclosed in the invention by providing a prolonged healing effect (maintaining dose).

A feature of the present invention is the correlation between the relative ratio of the "stabilized fraction" and the rate of hydrolysis of the cellulose-based carrier: At an oxidation degree of 2%, the adsorbed fraction is small, the "stabilized fraction" becomes disproportionately large and the "immobilized fraction" becomes too small to be therapeutically effective. In contrast, at an oxidation degree of 0.4%, most of the therapeutically active agent is bound to the cellulose-based carrier by adsorption only so that it can be rapidly washed out and the maintaining or minimum therapeutic dose that remains is not large enough to be therapeutically effective.

The optimal ratio between the three fractions as described above is achieved when a protocol for manufacturing a wound dressing according to this invention is implemented so that the reaction product comprises 0.4 mg of each protein per gram of cellulose-based carrier with a degree of maximal oxidation of approximately 0.9-1.1 %. This results in a shock dose of approximately 15%, a minimum therapeutic dose of approximately 15% and a maintaining dose of approximately 70%.

In summary, the high level of therapeutic efficiency of the therapeutically active agent is based on the formation of such a "triple system" and the subsequent dissociation of the therapeutically active agent from the fractions attached to the cellulose-based carrier upon contact with the wound. It is clear for those skilled in the art that this "triple system" can principally be established for any other protein to achieve the desired therapeutic effect.

The present invention is further illustrated by the following examples.

### Example 1: Production of dialdehyde cellulose

14 g of periodic acid is added to 2 L of distilled water in a glass vessel. In another glass vessel, 2.5 g of sodium hydroxide is solved in 2 L of distilled water. Both solutions are combined under agitation. A reactor with a capacity of 10 L is charged with 1 kg of medicinal gauze for use as carrier. The latter is exposed to the freshly prepared 4 L of sodium periodate for 15-20 hours at ambient temperature under intermittent agitation. The final product, dialdehyde cellulose (DAC) with a degree of maximal oxidation of 1.0 % ± 0.1%, is pressed to remove excess liquid, thoroughly washed with 5 x 10 L water and pressed again.

### Example 2: Immobilization of terrilytin and lysozyme or insulin

In a glass vessel 0.4 g of terrilytin is dissolved in 2 L of phosphate buffer pH 4.4 ± 0.4. Similarly, 0.4 g of lysozyme or insulin, respectively, is dissolved in 2 L of phosphate buffer pH 4.4 ± 0.4. The two solutions are mixed immediately prior to the immobilization process resulting in a final concentration of 0.1 g/L or 0.01 wt.-% each. The combined solutions are quickly added to a 10 L reactor which has been pre-loaded with 1 kg of activated cellulose-based carrier prepared as described in Example 1. After 4 hours at 20° Celsius the product is pressed to remove excess liquid until 1.5 wt.-% remaining moisture is achieved. The product is then incubated under refrigeration (4° Celsius) for another 4 hours. The treatment of DAC under the above conditions gives rise to a product in which virtually all of terrilytin and lysozyme or insulin becomes attached to said carrier (0.4 mg each per 1 g of DAC). The product is air dried until ≤ 10 % humidity is reached, cut into napkins and placed into polyethylene bags. Subsequently, the bags are sealed and sterilized with 25 kGy gamma irradiation.

### Example 3: Application of the wound dressing

90 female patients with chronic trophic ulcers on the back which are caused by a venous disorder were treated after a usual ulcer treatment with a wound dressing prepared according to Example 2 (46 patients with terrilytin and lysozyme; 44 patients with terrilytin and insulin). Said wound dressings were moistened with 0.5 % antiseptic solution, in particular chlorohexidine bigluconate solution, and placed on the wound for 2 days. In both cases clinical and cytological examinations revealed a complete cleaning of the wound from debris combined with commencing granulation and insular epithelisation.

## Claims

1. Wound dressing comprising a therapeutically active agent and a cellulose-based carrier, wherein the therapeutically active agent comprises the enzyme terrilytin or a derivative thereof and at least one additional protein is attached to said carrier and wherein said therapeutically active agent comprises at least a therapeutically active system comprising
a) an adsorbed fraction,
b) a stabilized fraction, and
c) an immobilized fraction
of said therapeutically active agent.

2. Wound dressing according to claim 1, wherein the additional protein is genetically engineered.

3. Wound dressing according to claim 1 or 2, wherein the additional protein is insulin.

4. Wound dressing according to claim 1 or 2, wherein the additional protein is lysozyme.

5. Wound dressing according to any one of claims 1-4 consisting of three fractions of therapeutically active agent.

6. Wound dressing according to any one of claims 1-5, wherein the proteins attached to said carrier consist of terrilytin, lysozyme and insulin.

7. Method for manufacturing a wound dressing comprising the steps of
- activating a cellulose-based carrier by oxidizing an initial medicinal gauze
- preparing separate protein solutions in buffered solutions;
- combining the prepared protein solutions to form a combined protein solution and adding the combined protein solution to said oxidized medicinal gauze or
- alternatively treating said oxidized medicinal gauze sequentially with separate protein solutions;
- allowing the protein solution to react for a period of time sufficient to reach a predetermined level of attachment;
- removing the final product from the protein solution, washing and drying it;
**characterized in that** the step of oxidizing results in a cellulose-based carrier having an oxidation degree of approximately 0.5-1.5 % and wherein terrilytin and insulin are attached to said activated carrier in equimolar proportions.

8. Method for manufacturing a wound dressing according to claim 7, wherein said step of oxidizing results in a degree of maximal oxidation of approximately 0.9-1.1 %.

9. Method for manufacturing a wound dressing according to claim 7, wherein the preparation of protein solutions comprises forming solutions each with a protein concentration in the range from 0.01 mg/ml to approximately 50 mg/ml each.

10. Method for manufacturing a wound dressing according to any one of claims 7-9, wherein the protein concentration is in the range from 0.1 mg/ml to approximately 2 mg/ml.

11. Method for manufacturing a wound dressing according to any one of claims 7-10, wherein the reaction product comprises 0.4 mg of each protein per gram of cellulose-based carrier which corresponds to
- 0.1 proteolytic unit for terrilytin/g of cellulose-based carrier;
- 10 U for lysozyme/g of cellulose-based carrier;
- 27 IU for insulin/g of cellulose-based carrier.

12. Method according to any one of claims 7-11, wherein terrilytin, lysozyme and insulin are attached to said activated carrier in equimolar proportions.

13. Wound dressing according any one of claims 1-6 for use for the treatment of pyo-necrotic wounds.

14. Use of the enzyme terrilytin for the manufacture of a medicament for the treatment of pyo-necrotic wounds, wherein the medicament is the wound dressing according to any one of claims 1-6.

## Patentansprüche

1. Wundverband umfassend ein therapeutisch aktives Agens und einen auf Cellulose basierenden Träger, wobei das therapeutisch aktive Agens das Enzym Terrilytin oder ein Derivat davon umfasst und mindestens ein zusätzliches Protein an dem Träger befestigt ist, und wobei das therapeutisch aktive Agens mindestens ein therapeutisch aktives System umfasst, welches
a) eine adsorbierte Fraktion,
b) eine stabilisierte Fraktion, und
c) eine immobilisierte Fraktion
des therapeutisch aktiven Agens umfasst.

2. Wundverband gemäß Anspruch 1, wobei das zusätzliche Protein gentechnisch manipuliert ist.

3. Wundverband gemäß Anspruch 1 oder 2, wobei dass das zusätzliche Protein Insulin ist.

4. Wundverband gemäß Anspruch 1 oder 2, wobei das zusätzliche Protein Lysozym ist.

5. Wundverband gemäß einem der Ansprüche 1 bis 4 bestehend aus drei Anteilen des therapeutisch aktiven Agens.

6. Wundverband gemäß einem der Ansprüche 1 bis 5, wobei die an dem Träger befestigten Proteine aus Terrilytin, Lysozym und Insulin bestehen.

7. Verfahren zur Herstellung eines Wundverbandes umfassend die Schritte
- Aktivieren eines auf Cellulose basierenden Trägers durch Oxidation eines medizinischen Gewebes;
- Herstellen von getrennten Protein-Lösungen in gepufferten Lösungen;
- Zusammenführen der hergestellten Protein-Lösungen zur Bildung einer kombinierten Protein-Lösung und Zugabe der kombinierten Protein-Lösung zu dem oxidierten medizinischen Gewebe oder
- alternativ sequentielle Behandlung des oxidierten medizinischen Gewebes mit getrennten Protein-Lösungen;
- Ermöglichen des Reagierens der Protein-Lösung für eine Zeitspanne, die ausreicht, um einen vorherbestimmten Grad der Befestigung zu erreichen;
- Entfernen des Endproduktes aus der Protein-Lösung, dessen Waschen und Trocknen;
**dadurch** charakterisiert, dass der Oxidationsschritt in einem auf Cellulose basierenden Träger mit einem Oxidationsgrad von ungefähr 0.5 - 1.5 % resultiert und wobei Terrilytin und Insulin an dem aktivierten Träger in äquimolaren Anteilen befestigt sind.

8. Verfahren zur Herstellung eines Wundverbandes gemäß Anspruch 7, wobei der Oxidationsschritt in einem maximalen Oxidationsgrad von ungefähr 0.9 - 1.1 % resultiert.

9. Verfahren zur Herstellung eines Wundverbandes gemäß Anspruch 7, wobei die Herstellung von Protein-Lösungen die Bildung von Lösungen umfasst, die jeweils eine Protein-Konzentration in dem Bereich von 0.01 mg/ml bis ungefähr 50 mg/ml haben.

10. Verfahren zur Herstellung eines Wundverbandes gemäß einem der Ansprüche 7 - 9, wobei die Protein-Konzentration in dem Bereich von 0.1 mg/ml bis ungefähr 2 mg/ml liegt.

11. Verfahren zur Herstellung eines Wundverbandes gemäß einem der Ansprüche 7 - 10, wobei das Reaktionsprodukt 0.4 mg von jedem Protein pro Gramm des auf Cellulose basierenden Trägers umfasst, welches
- 0.1 proteolytische Einheit von Terrilytin/g des auf Cellulose basierenden Trägers
- 10 U von Lysozym/g des auf Cellulose basierenden Trägers
- 27 IU von Insulin/g des auf Cellulose basierenden Trägers entspricht.

12. Verfahren gemäß einem der Ansprüche 7 - 11, wobei Terrilytin, Lysozym und Insulin an dem aktivierten Träger in äquimolaren Anteilen befestigt sind.

13. Wundverband gemäß einem der Ansprüche 1 - 6 zur Verwendung in der Behandlung von pyo-nekrotischen Wunden.

14. Verwendung des Enzyms Terrilytin zur Herstellung eines Medikamentes zur Behandlung von pyo-nekrotischen Wunden, wobei das Medikament der Wundverband gemäß einem der Ansprüche 1 - 6 ist.

## Revendications

1. Pansement comprenant un agent thérapeutiquement actif et un véhicule à base de cellulose, dans lequel l'agent thérapeutiquement actif comprend l'enzyme terrilytine ou un dérivé de celle-ci et au moins une protéine supplémentaire est attachée au dit véhicule et dans lequel ledit agent thérapeutiquement actif comprend au moins un système thérapeutiquement actif comprenant :
a) une fraction adsorbée,
b) une fraction stabilisée, et
c) une fraction immobilisée
dudit agent thérapeutiquement actif.

2. Pansement selon la revendication 1, dans lequel la protéine supplémentaire est génétiquement modifiée.

3. Pansement selon la revendication 1 ou 2, dans lequel la protéine supplémentaire est l'insuline.

4. Pansement selon la revendication 1 ou 2, dans lequel la protéine supplémentaire est le lysozyme.

5. Pansement selon l'une quelconque des revendications 1 à 4 se composant de trois fractions d'agent thérapeutiquement actif.

6. Pansement selon l'une quelconque des revendications 1 à 5, dans lequel les protéines attachées au dit véhicule consistent en la terrilytine, le lysozyme et l'insuline.

7. Procédé pour la fabrication d'un pansement comprenant les étapes de
- activer un véhicule à base de cellulose par oxydation d'une gaze médicale initiale ;
- préparer des solutions protéiniques séparées dans des solutions tamponnées ;
- combiner des solutions protéiniques préparées pour former une solution protéinique combinée et additionner de la solution protéinique combinée à ladite gaze médicale oxydée ou
- en variante, de traiter ladite gaze médicale oxydée séquentiellement avec des solutions protéiniques séparées ;
- laisser la solution protéinique réagir pendant une période suffisante pour atteindre un niveau prédéterminé d'attachement ;
- retirer le produit final de la solution protéinique, le laver et le sécher ;
**caractérisé en ce que** l'étape d'oxydation produit un véhicule à base de cellulose ayant un degré d'oxydation d'environ 0,5-1,5% et dans lequel la terrilytine et l'insuline sont attachées au dit véhicule activé dans des proportions équimolaires.

8. Procédé pour la fabrication d'un pansement selon la revendication 7, dans lequel ladite étape d'oxydation produit un degré d'oxydation maximale d'environ 0,9-1,1%.

9. Procédé pour la fabrication d'un pansement selon la revendication 7, dans lequel la préparation de solutions protéiniques comprend de former des solutions chacune avec une concentration de protéines dans l'intervalle de 0,01 mg/ml à environ 50 mg/ml chacune.

10. Procédé pour la fabrication d'un pansement selon l'une quelconque des revendications 7 à 9, dans lequel la concentration de protéines est dans l'intervalle de 0,1 mg/ml à environ 2 mg/ml.

11. Procédé pour la fabrication d'un pansement selon l'une quelconque des revendications 7 à 10, dans lequel le produit de réaction comprend 0,4 mg de chaque protéine par gramme de véhicule à base de cellulose, ce qui correspond à :
- 0,1 unité protéolytique de terrilytine/g de véhicule à base de cellulose ;
- 10 U de lysozyme/g de véhicule à base de cellulose ;
- 27 UI de insuline/g de véhicule à base de cellulose.

12. Procédé selon l'une quelconque des revendications 7 à 11, dans lequel la terrilytine, le lysozyme et l'insuline sont attachés au dit véhicule activé dans des proportions équimolaires.

13. Pansement selon l'une quelconque des revendications 1 à 6 à utiliser pour le traitement de blessures pyonécrotiques.

14. Utilisation de l'enzyme terrilytine pour la fabrication d'un médicament pour le traitement de blessures pyonécrotiques, dans laquelle le médicament est le pansement selon l'une quelconque des revendications 1 à 6.
